# EUROPÄISCHE PATENTSCHRIFT

(11) **EP 1 382 023 B1**
(45) Veröffentlichungstag und Bekanntmachung des Hinweises auf die Patenterteilung: **05.10.2005**
(21) Anmeldenummer: 02719636.9
(22) Anmeldetag: 20.02.2002
(51) Int. Cl.: G08B 21/00, A62B 9/00, A61B 5/00

(54) **ÜBERWACHUNGS- UND WARNSYSTEM FÜR UNTER GEFÄHRLICHEN EINSATZBEDINGUNGEN TÄTIGE PERSONEN**
MONITORING AND WARNING SYSTEM FOR INDIVIDUALS WORKING UNDER HAZARDOUS OPERATING CONDITIONS
SYSTEME DE SURVEILLANCE ET D'AVERTISSEMENT POUR DES PERSONNES TRAVAILLANT DANS DES CONDITIONS DANGEREUSES

(30) Priorität: 24.04.2001 DE 10120775
(43) Veröffentlichungstag der Anmeldung: 21.01.2004
(73) Patentinhaber: MSA Auer GmbH, 12059 Berlin (DE)
(72) Erfinder: SCHUBERT, Axel, 12309 Berlin (DE); LUBKOLL, Dieter, 12207 Berlin (DE)
(74) Vertreter: Wablat, Wolfgang
(86) Internationale Anmeldenummer: PCT/DE2002/000655
(87) Internationale Veröffentlichungsnummer: WO 2002/086834

(56) Entgegenhaltungen:
- DE-A- 10 008 048
- DE-A- 19 822 412
- US-A- 5 899 204

## Beschreibung

Die Erfindung betrifft ein Überwachungs- und Warnsystem für unter gefährlichen Einsatzbedingungen tätige Personen, mit einem vorzugsweise an einen Preßluftatmer angeschlossenen Überwachungsgerät (ICU), das aus einem Mikrocomputer sowie Meßwert- und Alarmanzeigen und Alarmgebern besteht, einem Telemetriemodul sowie weiteren Überwachungs- und/oder Steuergeräten.

Die Arbeit im Bergbau oder Tunnelbau, in einigen Bereichen der Industrie,im militärischen Bereich und bei der Bekämpfung von Bränden oder der Einsatz in durch Naturkatastrophen, Havarien und dergleichen kontaminierten Gebieten ist mit einer erheblichen Gefährdung der in dem betreffenden Bereich tätigen Personen verbunden. Derartige Gefährdungen bestehen beispielsweise im Auftreten von toxischen und/oder explosiven Gasen, gesundheitsschädlichen Strahlungen oder hohen Temperaturen. Gleichermaßen sind die Einsatzkräfte durch herabfallende Gegenstände und ähnliche physische Gewalteinwirkungen gefährdet oder durch starke körperliche Belastung in hohem Maße belastet. Zudem ist die Einsatzzeit der zumeist über Preßluftatmer mit Atemluft versorgten Personen durch den Preßluftflascheninhalt begrenzt und darf keineswegs überschritten werden.

Zur Überwachung der Einsatzbedingungen, zur Information vom Einsatzort, zur Kontrolle der Vitalfunktionen, zur Positionsbestimmung und zur Warnung der betreffenden Person bei gefährlichen oder gar lebensbedrohlichen Bedingungen oder zur Steuerung und Überwachung bestimmter Vorrichtungen, beispielsweise eines mit dem Preßluftatmer verbundenen Druckminderers oder eines Lungenautomaten, sind verschiedene, miteinander verbundene elektronische Warn-, Überwachungs-, Steuerungs- und Informationsgeräte (im weiteren Text auch kurz als Einheiten bezeichnet) erforderlich. Beispielsweise ist für den Einsatz von mit einem Preßluftatmer ausgerüsteten Feuerwehrleuten ein aus folgenden Einheiten bestehendes Überwachungs- und Warnsystem vorgesehen:
ein Überwachungsgerät (ICU) für den Preßluftatmer, das im wesentlichen Druck-, Bewegungs- und Temperatursensoren, Alarm- und Meßwertanzeigen (LED und LCD) sowie akustische Alarmgeber enthält;
ein Gaswarngerät für toxische und/oder explosive Gase
ein Positionserkennungsgerät (z.B. GPS)
ein Druckmeßgerät zur Pressluftflaschenmessung und -überwachung
Steuergeräte für den Druckminderer und den Lungenautomaten
ein Meßgerät zur Überwachung der Vitalfunktionen und
ein Informationssystem Kamera / Wärmebildkamera.

Über eine weitere wichtige Einheit des Gesamtsystems, ein Telemetriemodul, sollen der Status und die Daten der einzelnen Einheiten zu einer außerhalb des unmittelbaren Einsatzgebietes liegenden Basisstation abgesetzt werden, um auch von außen eine Kontrolle über die im Einsatzgebiet befindlichen Personen zu haben. Andererseits sollen von der Basisstation über bestimmte Einheiten Befehle, Informationen und Meldungen an die betreffende Person weitergeleitet werden.

Aus dem DE 19822412 ist ein System bekannt zur Verminderung der Gefahren für Atemschatzgeräteträger wobei kenndaten von Sensoren a Signalgebern zu einer Überwachungseinheit übertragen werden. Ein ähnliches system wird im DE 100 08 048 dargestellt.

Für derart umfassende elektronische Sicherheitssysteme, ist ein erheblicher Aufwand für die Kommunikation der Einheiten untereinander und mit dem Telemetriemodul sowie für das Einbinden neuer Komponenten in das Gesamtsystem erforderlich. Weiterhin sind die entsprechenden nationalen bzw. regionalen Standards/Vorschriften zu beachten. Darüber hinaus sind für die explosionsgeschützte und störungsfreie Ausführung und Einbindung der einzelnen Einheiten aufwendige zusätzliche Maßnahmen notwendig.

Der Erfindung liegt daher die Aufgabe zugrunde, ein mit einem Telemetriemodul zur bidirektionalen Kommunikation mit einer Basisstation ausgerüstetes Überwachungs- und Warnsystem für in gefährlichen Bereichen tätige Personen so auszubilden, daß bei einem einfachen und störungssicheren Verbindungsaufbau und einer einfachen Einbindung zusätzlicher Einheiten eine schnelle Datenübertragung zwischen den einzelnen Komponenten des Systems sowie von und zu der Basisstation gewährleistet ist.

Erfindungsgemäß wird die Aufgabe mit einem Überwachungs- und Warnsystem gemäß den Merkmalen des Patentanspruchs 1 gelöst.

Der Grundgedanke der Erfindung liegt dabei darin, daß bei einem solchen von der gefährdeten Person getragenen Gerätesystem sämtliche Überwachungs- und Warngeräte, Steuergeräte und das Telemetriemodul in einen gemeinsamen, offen oder geschlossen ausgeführten Bus integriert sind, wobei die Einheiten entweder für den Master-Slave-Betrieb oder für den Master-Master-Betrieb geschaltet sind. Im Master-Slave-Betrieb empfängt bzw. steuert eine der Einheiten die Informationen der restlichen Einheiten des Systems und gibt diese an das Telemetriemodul weiter. Im Master-Master-Betrieb mit gleichberechtigten Einheiten können die Einheiten untereinander einen Datenaustausch durchführen und jede einzelne Einheit kann ihre Informationen unmittelbar an das Telemetriemodul weiterleiten.

Ein derart ausgebildetes System kommt mit einem geringen Verkabelungs- und Anschlußaufwand aus. Zusätzliche Einheiten können problemlos in das Bus-System eingebunden werden. Der Status und die Meßdaten der einzelnen Einheiten können parallel und damit schnell zu einer abgesetzten Basisstation übertragen werden. Gleichzeitig können aber auch Meldungen und Befehle von der Basisstation zu den Einheiten gesandt werden. Die Ankopplung der Einheiten an den Bus kann so erfolgen, daß die Anforderungen an den Explosionsschutz und an einen störungsfreien Betrieb des Überwachungs- und Warnsystems erfüllt sind. Andererseits kann die Bus-Verbindung so aufgebaut werden, daß bei einer Leitungsunterbrechung die Übertragung der Informationen in der anderen Richtung erfolgen kann oder die Gefahr einer Leitungsunterbrechung überhaupt nicht besteht.

Aus den Unteransprüchen sowie der weiter unten folgenden Beschreibung einer bevorzugten Ausführungsform der Erfindung ergeben sich weitere Merkmale und zweckmäßige Weiterbildungen der Erfindung bzw. weitere Vorteile.

So kann der Bus entweder zur parallelen und damit schnellen Übertragung der Daten-, Adress- und Steuersignale oder zur seriellen Weiterleitung der Informationen mit dem Vorteil eines verminderten Verkabelungsaufwandes ausgebildet sein.

Nach einem weiteren Merkmal der Erfindung sind die einzelnen Einheiten galvanisch getrennt an den Bus angeschlossen und verfügen jeweils über eine eigene Stromversorgung. Dieser Lösungsvorschlag ist vorteilhaft für Überwachungs- und Warnsysteme geeignet, die in explosionsgefährdeten Bereichen zum Einsatz kommen sollen.

Andererseits kann aber auch bei einem nicht im EX-Bereich eingesetzten System eine zentrale Spannungsversorgung unmittelbar in den Bus eingebunden sein.

In vorteilhafter Weiterbildung der Erfindung können die Einheiten sternförmig angeordnet sein, wobei eine der Einheiten als Master fungiert und die Daten von den anderen Einheiten abfragt und zum Telemetriemodul überträgt, oder bei demgegenüber verringertem Kabelaufwand auch parallel an dem Bus liegen. Gemäß einer bevorzugten, sicherheitstechnisch vorteilhaften Ausführungsform ist ein serieller Ringbus vorgesehen, bei dem alle Informationen seriell von allen Einheiten übertragen werden und die Übertragung bei einer Unterbrechung des Busses auch in der anderen Richtung erfolgen kann. In weiterer Ausbildung der Erfindung ist schließlich auch eine hochfrequente Verbindung denkbar, bei der die Daten zwischen den Einheiten ohne Kabelverbinder ausgetauscht werden können und die Gefahr einer Leitungsunterbrechung nicht gegeben ist. Bei der HF-Verbindung ist ebenfalls der Master-Slave oder Master-Master Betrieb möglich.

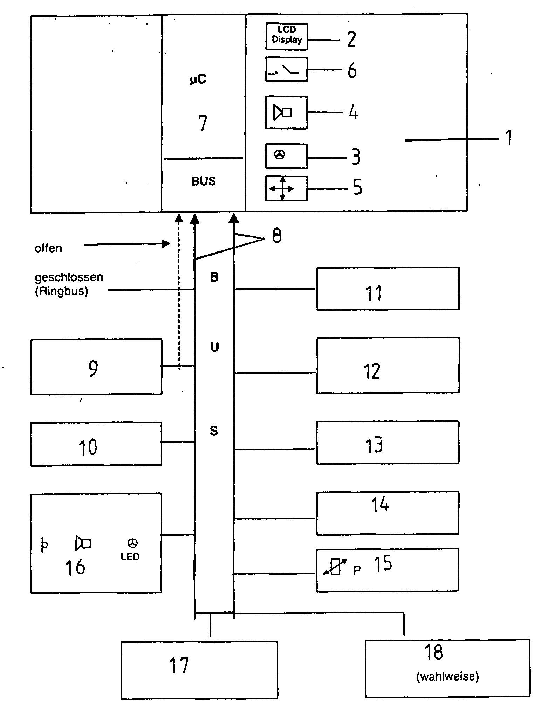

Ein Ausführungsbeispiel der Erfindung wird anhand der Zeichnung näher erläutert. In der einzigen Figur ist ein Blockschaltbild eines an einen Preßluftatmer (nicht dargestellt) angeschlossenen Überwachungs- und Warnsystems wiedergegeben ist, dessen einzelne Einheiten über einen Ringbus miteinander verbunden sind. Mit der strichlierten Darstellung in der Zeichnung wird angedeutet, daß der Bus auch offen ausgeführt sein kann.

Die Zeichnung zeigt ein mit einem Preßluftatmer verbundenes, als ICU bezeichnetes zentrales Überwachungsgerät 1, das über eine Meßwertanzeige (LCD) 2, eine Alarmanzeige (LED) 3, einen akustischen Alarmgeber 4, einen Bewegungsmelder 5 sowie Funktionstasten 6 und einen Mikrocomputer (µC) 7 verfügt. An einen geschlossenen Ringbus 8 sind neben dem Überwachungsgerät 1 eine erste Steuereinheit 9 für einen elektronischen Druckminderer des Preßluftatmers (nicht dargestellt), eine zweite Steuereinheit 10 für einen Lungenautomaten (nicht dargestellt), eine Meßeinheit 11 zur Überwachung der Vitalfunktionen der jeweiligen Person, ein Gaswarngerät 12 zur Detektion explosiver und/oder toxischer Gase, ein Positionserkennungsgerät 13 sowie eine Kamera 14, ein Drucksensor 15 zur Feststellung des Preßluftflaschendrucks, ein Helm-/Masken-Kommunikationsmodul 16 zur Informationsdarstellung im unmittelbaren Sichtbereich des Geräteträgers mit Hilfe eines Head-up-display sowie ein Telemetriemodul 17 zum Übertragen der Daten der einzelnen Einheiten an eine zentrale Basisstation (nicht dargestellt) bzw. zum Empfangen von Befehlen und Meldungen der Basisstation an die Einheiten bzw. den Geräteträger angeschlossen. Darüber hinaus kann wahlweise an dem Ringbus 8 eine zentrale Spannungsquelle 18 vorgesehen sein, um sämtliche Einheiten des Überwachungs- und Warnsystems mit der erforderlichen Energie zu versorgen. Diese Spannungsversorgung entfällt jedoch, wenn die jeweiligen, galvanisch getrennt an den Bus 8 angeschlossenen Einheiten eine eigene Spannungsquelle aufweisen, so daß ein Einsatz des Systems im EX-Bereich möglich ist. In ein derart über einen Bus 8 verbundenes Überwachungs- und Warnsystem können auch weitere Einheiten problemlos integriert werden.

Die einzelnen Einheiten bearbeiten ihre jeweiligen Aufgaben innerhalb des Gesamtsystems und geben die betreffenden Informationen an die zu steuernden Geräte, an die Alarm- und Meßwertanzeigen und die Alarmgeber in dem Überwachungsgerät (ICU) 1 oder in dem Helm-/Masken-Kommunikationsmodul 16 sowie - je nach Betriebsart - über eine Mastereinheit (z.B. die ICU 1) oder unmittelbar an das Telemetriemodul 17, um die Informationen der Überwachungs- und Warngeräte parallel zur Basisstation zu übertragen.

Bei der vorliegenden Verbindung der Einheiten über einen als Ringbus ausgeführten Bus 8 wird das System auch bei einer Unterbrechung der Bus-Leitung nicht außer Betrieb gesetzt, da die Informationsübertragung auch in der entgegengesetzten Richtung erfolgen kann.

In der Zeichnung wird durch die mit ? vorgenommene Kennzeichnung auf ein HF-Modell zur Kommunikation ohne Bus-verbindung (HF-Bus) hingewiesen, wobei die Stromversorgung kein HF-Modul benötigt, da sie mindestens mit einer Einheit verbunden ist, über die die Informationen der Spannungsquelle vorliegen.

### Bezugszeichenliste

- 1: Überwachungsgerät (ICU)
- 2: Meßwertanzeige (LCD) von 1
- 3: Alarmanzeige (LED) von 1
- 4: akustischer Alarmgeber von 1
- 5: Bewegungsmelder von 1
- 6: Funktionstasten von 1
- 7: Mikroprozessor (µC) von 1
- 8: Bus (Ringbus)
- 9: erste Steuereinheit (für Druckminderer)
- 10: zweite Steuereinheit (für Lungenautomat)
- 11: Meßeinheit (Vitalfunktionen)
- 12: Gaswarngerät (explosive und toxische Gase)
- 13: Positionserkennungsgerät (PSB)
- 14: Kamera
- 15: Drucksensor (Preßluftflaschendruck)
- 16: Helm-/Masken-Kommunikationsmodul/head-up display
- 17: Telemetriemodul
- 18: Spannungsquelle

## Patentansprüche

1. Überwachungs- und Warnsystem für im Brand- und Katastropheneinsatz tätige Rettungskräfte, das ein mit einem Pressluftatmer verbundenes Überwachungsgerät (1) mit Mikrocomputer, Messwert- und Alarmanzeigen, Alarmgebern (4) und Bewegungsmelder (5) sowie ein mit einer Basisstation bidirektional verbundenes Telemetriemodul (17) umfasst, **gekennzeichnet durch** ein der betreffenden Rettungskraft zugeordnetes geschlossenes oder offenes Bussystem (8), an das das Überwachungsgerät (1) und das Telemetriemodul (17) sowie wahlweise und austauschbar eine Mehrzahl zusätzlicher Überwachungs-, Kommunikations- und Steuermodule (9-16) galvanisch mit zentrale Energieversorgung und/oder jeweils mit eigener Energieversorgung galvanisch getrennt über eine optische und/oder eine Funkverbindung angeschlossen sind.

2. Überwachungs- und Warnsystem nach Anspruch 1, **gekennzeichnet durch** einen Bus (8) mit paralleler Übertragung der Daten-, Adress- und Steuersignale.

3. Überwachungs- und Warnsystem nach Anspruch 1, **gekennzeichnet durch** einen seriellen Bus (8) zur seriellen Übertragung der Daten-, Adress- und Steuersignale in demselben Leitungsstrang.

4. Überwachungs- und Warnsystem nach einem der Ansprüche 1 bis 3, **dadurch gekennzeichnet, daß** die Verbindung der Einheiten sternförmig ist, und zwar mit einer der Einheiten als Bus-Master für das Abfragen der jeweiligen Daten von den anderen Einheiten und die Übertragung dieser Daten zum Telemetriemodul (17).

5. Überwachungs- und Warnsystem nach einem der Ansprüche 1 bis 4, **dadurch gekennzeichnet, daß** die einzelnen Einheiten des Systems seriell in einen Ringbus (8) eingebunden sind.

## Claims

1. A monitoring and warning system for rescue personnel fighting fires and responding to disasters that includes a monitoring device (1) connected to a compressed air breathing apparatus with a microcomputer, displays for measured values and alarms, alarm transmitters (4), a motion detector (5) as well as a telemetric module (17) having a bidirectional connection to a base station, **characterized in that** it copmprises a closed-type or open-type bus system (8) assigned to the respective rescuer to which the monitoring device (1) and the telemetric module (17) and, optionally and exchangeably, a multitude of additionmal monitoring, communication, and control modules (9-16) can be connected, either galvanically with a central power supply unit and/or with their own galvanically isolated power supply via an optical and/or radio connection.

2. The monitoring and warning system according to claim 1, **characterized in that** it comprises a bus (8) with parallel transfer of data, address and control signals.

3. The monitoring and warning system according to claim 1, **characterized in that** it comprises a serial bus (8) for serial transfer of data, address and control signals over one and the same line section.

4. The monitoring and warning system according to any one of claims 1 through 3, **characterized in that** the units are connected in a star shape wherein one of the units operates as bus master for scanning the respective data from the other units and transferring this data to the telemetric module (17).

5. The monitoring and warning system according to any one of claims 1 through 4, **characterized in that** the units of the system are connected in series to a ring bus (8).

## Revendications

1. Système de surveillance et d'avertissement pour des sauveteurs travaillant au cours d'interventions en cas d'incendie et de catastrophes, comprenant un appareil de surveillance (1) relié à un respirateur à air comprimé avec microordinateur, indicateurs de mesure et d'alarme, postes émetteurs d'alarme (4) et détecteur de mouvements (5) ainsi qu'un module de télémétrie (17) relié de manière bidirectionnelle à une station de base, **caractérisé par** un système de bus (8) fermé ou ouvert et associé au sauveteur concerné, auquel l'appareil de surveillance (1) et le module de télémétrie (17) ainsi que, au choix et de manière interchangeable, une multitude de modules additionnels de surveillance, de communication et de commande (9 à 16) sont connectés par voie galvanique à une alimentation en énergie centrale et/ou chacun séparément par voie galvanique à une propre alimentation en énergie via une liaison optique et/ou une liaison radio.

2. Système de surveillance et d'avertissement selon la revendication 1, **caractérisé par** un bus (8) avec transmission parallèle des signaux de données, d'adresses et de commande.

3. Système de surveillance et d'avertissement selon la revendication 1, **caractérisé par** un bus (8) série pour la transmission sérielle des signaux de données, d'adresses et de commande dans le même faisceau de lignes.

4. Système de surveillance et d'avertissement selon l'une des revendications 1 à 3, **caractérisé en ce que** la liaison des unités est en forme d'étoile, et plus précisément avec une des unités en tant que bus maître pour l'interrogation des données respectives des autres unités et pour la transmission de ces données au module de télémétrie (17).

5. Système de surveillance et d'avertissement selon l'une des revendications 1 à 4, **caractérisé en ce que** les unités individuelles du système sont rassemblées en série dans un bus en anneau (8).
